# EUROPEAN PATENT SPECIFICATION

(11) **EP 3 724 357 B1**
(45) Date of publication and mention of the grant of the patent: **03.01.2024**
(21) Application number: 18826108.5
(22) Date of filing: 30.11.2018
(51) Int. Cl.: C12Q 1/6886, C07K 14/435, G01N 33/574

(54) **NEW ONCO-IMMUNOLOGIC PROGNOSTIC AND THERANOSTIC MARKERS**
NEUE ONKO-IMMUNOLOGISCHE PROGNOSTISCHE UND THERANOSTISCHE MARKER
NOUVEAUX MARQUEURS PRONOSTIQUES ET THÉRANOSTIQUES ONCO-IMMUNOLOGIQUES

(30) Priority: 11.12.2017 IT 201700142293
(43) Date of publication of application: 21.10.2020
(73) Proprietor: Istituti Fisioterapici Ospitalieri (IFO) - Istituto Regina Elena Per Lo Studio E La Cura Dei Tumori, 00144 Roma (IT)
(72) Inventor: NISTICO', Paola, 00144 Roma (IT); DI MODUGNO, Francesca, 00144 Roma (IT)
(74) Representative: Predazzi, Valentina
(86) International application number: PCT/IB2018/059499
(87) International publication number: WO 2019/116146

(56) References cited:
- WO-A1-2012/020281
- PIETRO FICI ET AL: "Splicing factor ratio as an index of epithelial-mesenchymal transition and tumor aggressiveness in breast cancer", ONCOTARGET, vol. 8, no. 2, 10 January 2017 (2017-01-10), pages 2423-2436, XP055471635, DOI: 10.18632/oncotarget.13682
- ROBERTA MELCHIONNA ET AL: "The pattern of hMENA isoforms is regulated by TGF-[beta]1 in pancreatic cancer and may predict patient outcome", ONCOIMMUNOLOGY, vol. 5, no. 12, 12 August 2016 (2016-08-12), page e1221556, XP055471633, DOI: 10.1080/2162402X.2016.1221556 cited in the application
- MICHELE BALSAMO ET AL: "The alternatively-included 11a sequence modifies the effects of Mena on actin cytoskeletal organization and cell behavior", SCIENTIFIC REPORTS, vol. 6, no. 1, 17 October 2016 (2016-10-17), XP055471636, DOI: 10.1038/srep35298
- SISTIGU ANTONELLA ET AL: "Deciphering the loop of epithelial-mesenchymal transition, inflammatory cytokines and cancer immunoediting", CYTOKINE AND GROWTH FACTOR REVIEWS, ELSEVIER LTD, GB, vol. 36, 31 May 2017 (2017-05-31), pages 67-77, XP085177633, ISSN: 1359-6101, DOI: 10.1016/J.CYTOGFR.2017.05.008
- EMILIO BRIA ET AL: "Prognostic impact of alternative splicing-derived hMENA isoforms in resected, node-negative, non-small-cell lung cancer", ONCOTARGET, vol. 5, no. 22, 30 November 2014 (2014-11-30), XP055471637, United States ISSN: 1949-2553, DOI: 10.18632/oncotarget.2609 cited in the application

## Description

The present invention relates to the identification of a set of prognostic and theranostic markers enabling to assess the tumor microenvironment (immunogenic or immunosuppressive) of solid tumors and predict the responsiveness or the non-responsiveness to immunotherapy of the affected patient.

### STATE OF THE PRIOR ART

The identification of therapeutic response biomarkers and the development of novel therapeutic strategies targeting signal pathways involved in resistance mechanisms, to be employed in combined therapies, represent fundamental objectives in oncology, and in particular in the new era of tumor immunotherapy.

Solid tumors contain various non-tumor cell types, among which fibroblasts and immune system cells (or "immune cells") and soluble factors, immersed in an extracellular matrix of which fibronectin is a relevant factor, that, in dynamic reciprocity with tumor cells, make up the tumor microenvironment (TME). The various microenvironments favor or prevent tumor progression and influence response to therapy (Hirata E, Sahai E. Tumor Microenvironment and Differential Responses to Therapy. Cold Spring Harb Perspect Med. 2017;7(7); Chen DS, Mellman I. Elements of cancer immunity and the cancer-immune set point. Nature. 2017 Jan 18;541(7637):321-330). Tumor and stromal cells co-evolve during tumorigenesis and tumor progression, and their interaction causes phenotypic and functional plasticity, which may generate tissue architecture alteration, alteration of extracellular matrix (ECM) geometry and stiffness, and the entailed alteration of immune cell localization and functionality. ECM stiffness is one of the mechanisms influencing immune cell recruiting inside the tumor. On the other hand, the immune contexture of a tumor is considered a prognostic factor in various solid tumors (Galon J. et al Type, density, and location of immune cells within human colorectal tumors predict clinical outcome. Science. 2006 Sep 29;313(5795):1960-4.; Fridman WH, Zitvogel L, Sautès-Fridman C, Kroemer G. The immune contexture in cancer prognosis and treatment. Nat Rev Clin Oncol. 2017 Dec;14(12):717-734.). Moreover, in-tumor neoformation of lymph node-like structures termed tertiary lymphoid structures (TLS) was indicated as a positive prognostic factor in various solid tumors, and in particular in non-small cell lung cancers (NSCLCs) (Sautès-Fridman et al Tertiary Lymphoid Structures in Cancers: Prognostic Value, Regulation, and Manipulation for Therapeutic Intervention. Front Immunol. 2016 Oct 3; 7:407. eCollection 2016).

The role of the host immune system in neoplastic disease control and in the response to various treatments such as chemo-, radio- and target therapy is by now a widely established concept. Recently, immunotherapy has become a first-line therapy in various solid tumors, and, considering response effectiveness and durability, antibodies' use towards immune (immunological) checkpoint inhibitors was approved for treatment of various tumor types (Shields BD, Mahmoud F, Taylor EM, Byrum SD, Sengupta D1 Koss B, Baldini G, Ransom S, Cline K, Mackintosh SG, Edmondson RD, Shalin S, Tackett AJ Indicators of responsiveness to immune checkpoint inhibitors., Sci Rep. 2017 Apr 1 1;7(1):807)

However, only a portion of patients responds to said therapies, which have extremely high costs. Hence, the urgent need to identify biomarkers of response to such drugs.

Tumors have been classified relative to type, density, location and functionality of immune cell infiltrate, which can represent a prognostic and predictive factor in various solid tumors, as has been demonstrated above all in colorectal carcinoma (Galon, Science 2016 above). Recently, said classification has been integrated by new data indicating that tumors that lose the epithelial characteristics, modifying into mesenchymal and/or enriching in mesenchymal traits both in tumor cells and in the stromal component (mainly consisting of fibroblasts) are related to unfavorable prognosis (Becht E Immune and Stromal Classification of Colorectal Cancer Is Associated with Molecular Subtypes and Relevant for Precision Immunotherapy. Clin Cancer Res. 2016 Aug 15;22(16):4057-66). Hence, the need to integrate immune cell infiltrate assessment with an assessment of the "epithelial" or "mesenchymal" characteristics of tumor cells and stromal components consisting of tumor-associated fibroblasts. On the other hand, every tumor onsets and progresses in its own microenvironment, which conditions, besides tumor cells properties, the recruiting, spatial organization and function of the various immune cell populations.

Under the selective pressure of the immune system, tumor cells adopt a set of strategies for losing their immunogenicity, among which epithelial-mesenchymal transition (EMT), a physiological program during embryonal development, which once activated in tumor cells alters cell-cell adhesion, enables cell migration, confers mesenchymal traits and induces resistance to therapy (Nisticò P, Bissell MJ, Radisky DC. Epithelial-mesenchymal transition: general principles and pathological relevance with special emphasis on the role of matrix metalloproteinases. Cold Spring Harb Perspect Biol. 2012 Feb 1;4(2); Shibue T, Weinberg RA. EMT, CSCs, and drug resistance: the mechanistic link and clinical implications. Nat Rev Clin Oncol. 2017;14(10):611-29;). Recently, it was suggested that tumor subtypes with mesenchymal characteristics exhibit an immunosuppressive tumor microenvironment (Becht 2016, above). Of theranostic relevance, the expression of genes related to mesenchymal characteristics in the tumor, including E-Cadherin downregulation, has been associated with resistance to immune (immunological) checkpoint inhibitors such as the PD-1/ PD-L1 pathway (Hugo W et al. Genomic and Transcriptomic Features of Response to Anti-PD-1 Therapy in Metastatic Melanoma. Cell. 2016; 165(1):35-44), a novel therapeutic reality in various solid tumors. As mentioned above, immunotherapy protocols with anti-PD-1 and anti-PD-L1 antibodies were approved for treatment of various tumor types, considering response effectiveness and durability, but only a portion of patients responds to such therapies. Currently, the sole marker of response used in clinical practice is PD-L1 expression in the tumor, despite it being evident that the complexity of immune response may not be predicted by a single marker, and in fact that not all tumor PD-L1-positive patients respond, and some PD-L1-negative patients have instead benefited from these treatments. It has been suggested that individuals with a T-lymphocyte response pre-existing to therapy may benefit from immune checkpoint inhibitor blocking treatments, whereas resistance is associated with poor T lymphocyte infiltration (Chen, 2017, above) and an enrichment of mesenchymal traits (Hugo W, 2017, above). To date, validated biomarkers of mesenchymal "signatures" are yet to be defined.

hMENA, identified as antitumor antigen by SEREX technology, belongs to the ENA/VASP family, proteins regulating cell signaling pathways, and that depend on actin cytoskeleton dynamics. hMENA is undetectable in healthy tissues but is overexpressed in breast (Di Modugno et al 2006), lung (Bria et al 2014), pancreatic (Melchionna et al. 2014), gastric (Gurzu S, Kadar Z, Sugimura H, Bara T, Bara T Jr, Halmaciu I, Jung I. Gastric cancer in young vs old Romanian patients: immunoprofile with emphasis on maspin and mena protein reactivity. APMIS. 2015 Mar;123(3):223-33.), liver (Hu K, Wang J, Yao Z, Liu B, Lin Y, Liu L, Xu L. Expression of cytoskeleton regulatory protein Mena in human hepatocellular carcinoma and its prognostic significance. Med Oncol. 2014 May;31(5):939), salivary gland (Gurzu S, Krause M, Ember I, Azamfirei L, Gobel G, Feher K, Jung I. Mena, a new available marker in tumors of salivary glands? Eur J Histochem. 2012 Feb 7;56(1):e8), cervical (Gurzu S, Jung I, Prantner I, Chira L, Ember I. The immunohistochemical aspects of protein Mena in cervical lesions. Rom J Morphol Embryol. 2009;50(2):213-6) and colorectal (Gurzu S, Jung I, Prantner I, Ember I, Pávai Z, Mezei T. The expression of cytoskeleton regulatory protein Mena in colorectal lesions. Rom J Morphol Embryol. 2008;49(3):345-9. PubMed PMID: 18758639) tumors, and its alternative splicing generates various isoforms. Two tissue-specific isoforms, hMENA11a and hMENAΔv6, were identified, associated with an epithelial or mesenchymal phenotype respectively, and with an opposite role in cell proliferation and invasion. hMENA11a is expressed in epithelial phenotype cells which express the main component of adherens junctions, E-cadherin, whereas hMENAΔv6 is only present in tumor cells with migration ability and invasive phenotype that do not express E-cadherin and hMENA11a, while expressing mesenchymal markers such as N-cadherin and vimentin (Di Modugno F. et al Splicing program of human MENA produces a previously undescribed isoform associated with invasive, mesenchymal-like breast tumors. Proc Natl Acad Sci U S A. 2012 Nov 20; 109(47): 19280-5; Bria E, et al Prognostic impact of alternative splicing-derived hMENA isoforms in resected, node-negative, non-small-cell lung cancer. Oncotarget. 2014 Nov30;5(22):11054-63; Melchionna R. et al The pattern of hMENA isoforms is regulated by TGF-β1 in pancreatic cancer and may predict patient outcome. Oncoimmunology. 2016 Aug 12;5(12): e1221556). In early node-negative NSCLC patients, hMENA11a expression in connection with hMENAΔv6 expression represents a powerful prognostic factor for accurately predicting the risk of disease relapse (Bria E, 2014). Accordingly, in pancreatic adenocarcinoma tissues hMENA11a expression correlates with higher patient survival, with respect to tumors expressing low levels of 11a and high levels of hMENA/hMENAΔv6 (Melchionna R, 2016, above). Moreover, it was demonstrated that the hMENA gene is regulated by pathways involved in an immunosuppressive tumor microenvironment, such as b-Catenin and TGFβ. TGFβ, one of the major immunosuppressive cytokines, upregulates hMENA and hMENAΔv6, and downregulates hMENA11a. Of relevance, the silencing of all hMENA isoforms in hMENAΔv6-expressing cell lines inhibits TGF-β1 signaling pathways and TGFβ-mediated epithelial-mesenchymal transition (EMT) (Melchionna, 2016, above), suggesting a crucial role of hMENA/hMENAΔv6 in EMT-related tumor cell invasion processes. On the contrary, hMENA11a expression in breast carcinoma tumor cells correlates with a reduced expression of colony-stimulating factor 1 and inhibits tumor cells ability to invade and intravasate into blood vessels, suggesting an important anti-invasive role of the 11a isoform of hMENA (Roussos ET, Balsamo M, Alford SK, Wyckoff JB, Gligorijevic B, Wang Y, Pozzuto M, Stobezki R, Goswami S, Segall JE, Lauffenburger DA, Bresnick AR, Gertler FB,Condeelis JS. Mena invasive (MenaINV) promotes multicellular streaming motility and transendothelial migration in a mouse model of breast cancer. J Cell Sci. 2011 Jul 1;124(Pt 13):2120-31.)

The research paper Pietro Fici ET AL: "Splicing factor ratio as an index of epithelial mesenchymal transition and tumor aggressiveness in breast cancer", Oncotarget, vol. 8, no. 2, 10 January 2017, pages 2423-2436; discloses an assay to identify a new early prognostic marker of metastasis. Results showed that an increased expression of FN1 in cancer cells was reported.

The scientific report Michele Balsamo ET AL: "The alternatively-included 11a sequence modifies the effects of Mena on actin cytoskeletal organization and cell behavior", Scientific Reports, vol. 6. no. 1, 17 October 2016; discloses that RNA seq data analysis from patient cohorts demonstrates that the difference between mRNAs encoding constitutive Mena sequences and those containing the 11a exon correlates with metastasis in colorectal cancer, suggesting that 11a exon exclusion contributes to invasive phenotypes and leads to poor clinical outcomes.

The PCT patent application WO 2012/020281 A1 provides the use of a new hMena splicing variant, for diagnostic and medical purposes, wherein the expression of said variant is analysed in combination with the hMena 1 expression.

### SUMMARY OF THE INVENTION

Object of the present invention is a method useful for defining a microenvironment, immunogenic or immunosuppressive, of a tumor as prognostic and theranostic factor in solid tumors, by assessing the expression pattern of hMENA isoforms, (hMENA11a and, optionally, hMENAΔv6), both in tumor cells and in the stroma, in combination with the expression of fibronectin 1, PD-L1 and the CD3, CD8 and CD20 markers, for the localization of any lymphocyte infiltrate of T and B lymphocytes, even organized in tertiary lymphoid structures consisting of an aggregate of B lymphocytes within the structure and T cells outside the same, assessed respectively by the expression of CD20 and CD3 markers.

The Authors of the present invention have in fact discovered that the assessing, in bioptic samples of solid tumors, of the values of the expression of hMena isoforms, in particular of 11a isoform, in combination with data on the expression of other markers, indicated above and described in detail hereinafter, enables to identify the immunogenic or immunosuppressive characteristics of the tumor microenvironment of said tumors, thereby enabling to have a predictive information on the responsiveness of patients from which said samples originate to immunotherapy, optionally combined with other therapies.

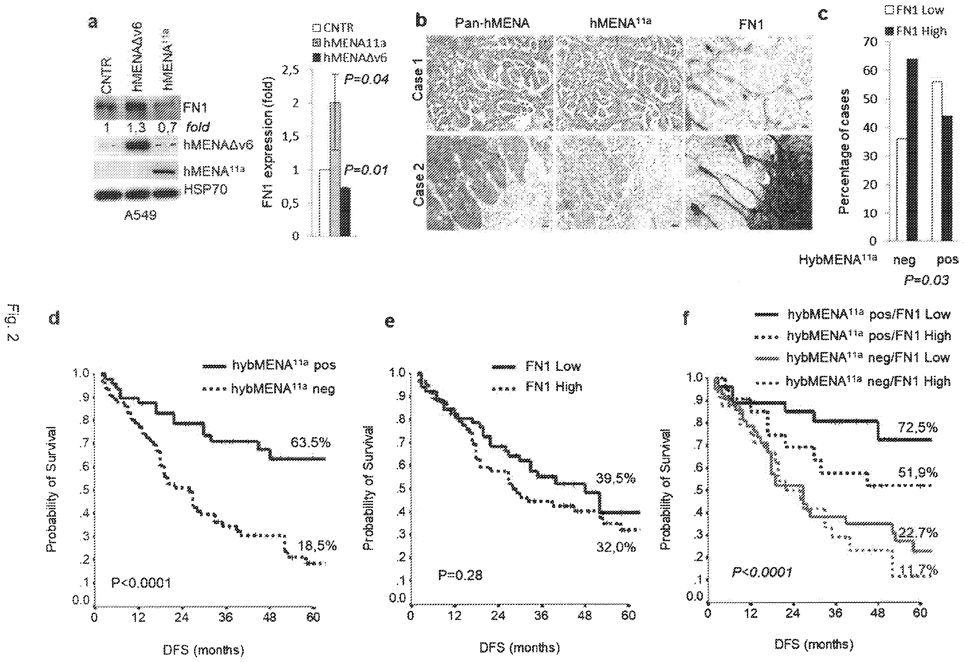

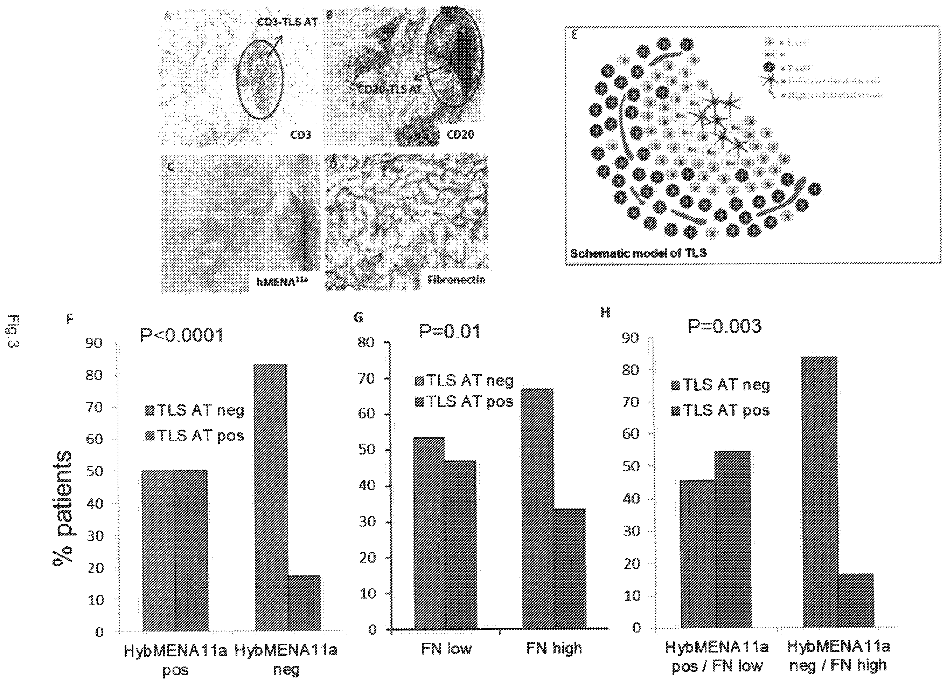

In the present invention, the Authors propose the assessment of the expression of the 11a isoform of hMENA, and optionally of Δv6 isoform of hMENA, as 1) EMT markers, 2) markers of an immunogenic microenvironment in the presence of hMENA11a in tumor cells and low expression of fibronectin in the stroma associated with lymphocyte infiltrate organized in tertiary lymphoid structures in the tumor area (Figure 3), identifying patients with a better prognosis (Figure 2), 3) or as markers of an immunosuppressive environment, in the absence of hMENA11a (and presence of hMENAΔv6) in tumor cells and high expression of fibronectin in the stroma and absence of lymphocyte infiltrate organized in tertiary lymphoid structures (Figure 3). The data disclosed in the present description indicate that a method based on hMENA isoform expression and on some characteristics of the tumor microenvironment, including lymphocyte infiltrate, can be defined to identify patients responsive or resistant to immunomodulatory treatments, and in particular to immune checkpoint inhibitors. The data presented herein and their prognostic and theranostic application are supported by recent literature data summarized in Table 1 below, identifying resistance signatures linked to mesenchymal traits.

**Table 1**

| **Table 1. Intrinsic tumor factors linked to resistance to immune checkpoint inhibitors** | | | |
|---|---|---|---|
| **Intrinsic factors** | **Mechanism** | **Effects** | **Refs** |
| β-catenin pathway | CCL4 transcription inhibition by ATF3 transcription repressor increase | Loss of CD103+ dendritic cells, T lymphocyte exclusion | Spranger S, Bao R, Gajewski TF. Melanoma-intrinsic beta-catenin signalling prevents anti-tumour immunity. Nature. 2015;523(7559):231-5. |
| Axl pathway | Antigen presentation inhibition, immunosuppressive cytokine production | Anti-tumor immune response inhibition, T lymphocyte exclusion | Aguilera TA, Giaccia AJ. Molecular Pathways: Oncologic Pathways and Their Role in T-cell Exclusion and Immune Evasion-A New Role for the AXL Receptor Tyrosine Kinase. Clin Cancer Res. 2017;23(12):2928-33. |
| PTEN loss | Immunosuppressive cytokine (i.e. VEGF) increase | Immunosuppressive cell (immature dendritic cells, MDSCs and regulatory cell) recruitment | Moens S, Goveia J, Stapor PC, Cantelmo T AR, Carmeliet P. The multifaceted activity of VEGF in angiogenesis - Implications for therapy responses. Cytokine Growth Factor Rev. 2014;25(4):473-82. |
| p53 activity | • DD1α, PD-1 and PD-L1 increase on tumor cells • PD-L1 regulation via miR-34 | T lymphocyte-mediated immune response suppression | Munoz-Fontela C, Mandinova A, Aaronson SA, Lee SW. Emerging roles of p53 and other tumoursuppressor genes in immune regulation. Nat Rev Immunol. 2016;16(12):741-50. Cortez MA, Ivan C, Valdecanas D, Wang X, Peltier HJ, Ye Y, et al. PDL1 Regulation by p53 via miR-34. J Natl Cancer Inst. 2016;108(1).92] |

| | | | |
|---|---|---|---|
| CCL4: C-C motive ligand 4 chemokine. ATF3: cyclic AMP-dependent transcription factor 3. PTEN: phosphatase and tensin homolog. VEGF: vascular endothelial growth factor. MDSC: myeloid-derived suppressor cell. DD1α: Death Domain 1α. PD-1: Programmed cell Death-1. PD-L1: Programmed cell Death-ligand 1. | | | |

In a recent study by Hugo et al. 2016, cited above, analyzing biopsies from advancedstage melanoma patients treated with anti-PD-1 antibodies, a panel of genes correlated to mesenchymal characteristics, upregulated in patients non-responsive to therapy with respect to responsive patients, is described. E-Cadherin negative regulation, and metalloproteinase positive regulation are also included among the genes, suggesting that the mesenchymal traits of the primitive tumor may be associated with an innate resistance to anti-PD-1. Moreover, resistance signatures demonstrate that β-catenin and Axl pathways (see Table 1 above) are strictly correlated with resistance to immunotherapy. Said data support the practical application described herein of experimental data reported in the present description, indicating that the absence of hMENA11a expression (and hMENAΔv6 expression) are correlated with the absence of E-Cadherin and a higher metalloproteinase activity.

The Authors of the invention have also considered the stroma surrounding tumor cells, which, with the presence of tumor-associated fibroblasts (CAFs), provides a specialized microenvironment with an important role in resistance to treatments and to immune response.

In colon tumor, Becht and coll. were able to identify a "mesenchymal/stromal" subtype characterized by an immunosuppressive "signature" correlated with a high density of fibroblasts able to produce chemokines, cytokines and angiogenic and proinflammatory factors (Becht, 2016). CAFs represent the main source of deposition of extracellular matrix, whose composition and stiffness constitute one of the main obstacles to immune cell infiltration (Salmon H, 2012). The Authors of the present invention have discovered that CAFs do not express hMENA11a, which is never expressed in tumor-surrounding stroma, whereas they express the hMENAΔv6 isoform inducing an activation of tumor-associated fibroblasts.

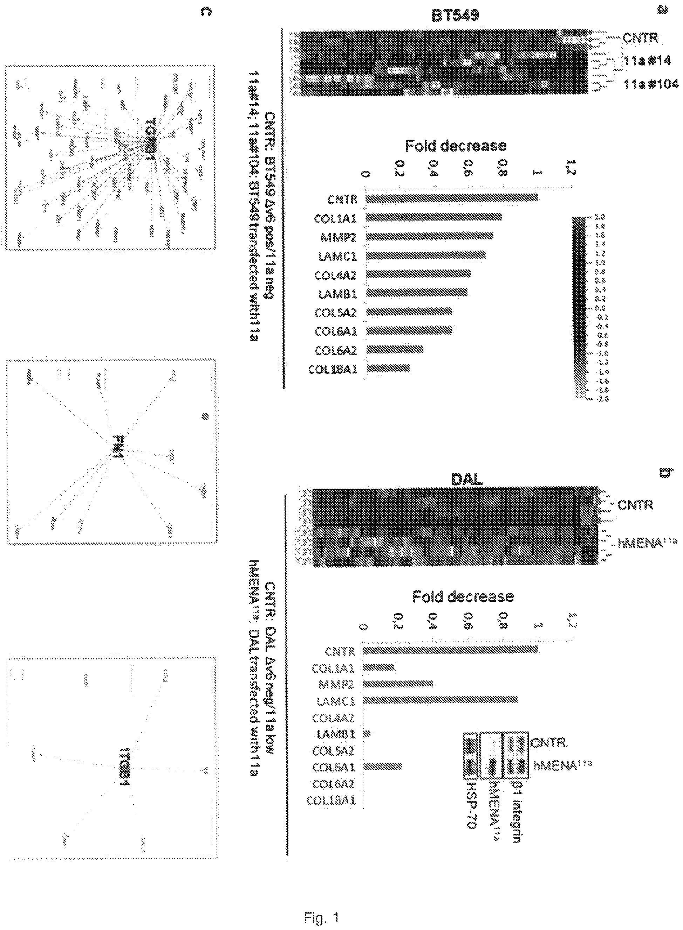

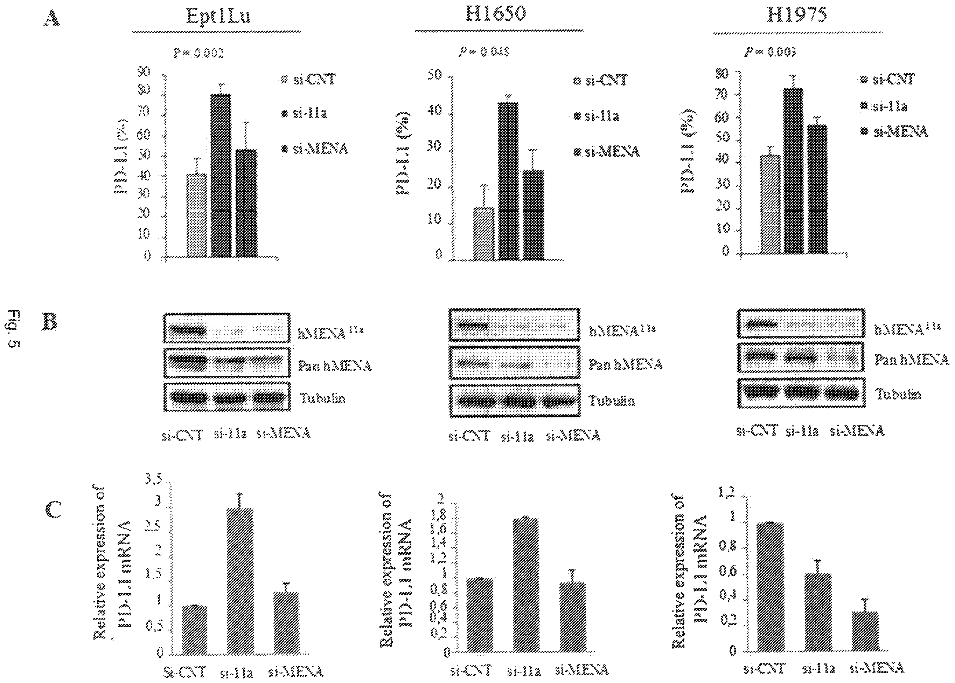

On the other hand, hMENA11a presence on tumor cells reduces the production of extracellular matrix components, altering their composition and fostering a proimmunogenic microenvironment (Figure 1). As a result, the expression of hMENA11a in parallel with the presence of lymphocytes also organized in tertiary lymphoid structures represent a marker of sensitivity. On the other hand, the presence of a lymphocyte infiltrate in the tumor was correlated with the response to immune checkpoint inhibitors. The Authors of the invention have discovered that in tumor tissues of early NSCLC patients, hMENA11a presence is associated with a low expression of fibronectin in the stroma, and that these characteristics identify patients with better prognosis and are associated with the organization of tertiary lymphoid structures in the tumor area. Grade, amount and spatial distribution of the lymphocyte infiltrate represent a marker of clinical response to immunotherapies (Galon, 2006) and recently it was demonstrated that the induction of tertiary lymphoid structures increases the effectiveness of immune checkpoint inhibition (Percival AJ 2017). Preliminary data obtained by the Authors of the invention, derived from an RNA-SEQ study on a lung carcinoma cell line (H1650) silenced for hMENA11a, indicate a significant reduction (p=0.0004) of LTBR, an important receptor for the organization of tertiary lymphoid structures, with respect to control cells. Moreover, the data obtained by the Authors of the invention indicate that hMENA11a silencing induces in tumor cell lines an increase in PDL-1 expression (Figure 5).

Currently, immunohistochemistry-assessed PDL-1 expression is the sole selection criterion in clinical practice for patients' enrolment to immunotherapy protocols with the PD-1-PDL1 pathway as target. Despite PD-L1 expression having been correlated to a better prognosis, evidently a single biomarker cannot represent the complexity of the interactions in a tumor microenvironment.

In light of the experimental data, and of those obtained in tissues from lung carcinoma patients, the Authors of the invention have discovered that a tumor with an immunogenic microenvironment responsive to therapies with immune checkpoint inhibitors expresses (assessable by immunohistochemistry in paraffin-included tumor tissues): 1) hMENA11a in the tumor component, 2) low fibronectin expression in the stroma, 3) CD3, CD8, CD20 lymphocyte infiltrate localized inside the tumor area (AT), also organized in tertiary lymphoid structures (TLS), 4) absence of expression of hMENA in the stroma (absence of CAF), 5) non-high levels of PD-L1. On the contrary, a tumor with an immunosuppressive microenvironment resistant to therapies with immune checkpoint inhibitors: 1) expresses low levels of hMENA11a 2) exhibits high fibronectin expression in the stroma, 3) does not show CD3, CD8, CD20 lymphocyte infiltrate 4) expresses hMENA/hMENAΔv6 in the stroma (presence of pro-invasive activated CAFs), 5) can express different levels of PD-L1.

Therefore, objects of the invention are:
an assay to assess in a tumor the microenvironment, immunogenic or immunosuppressive, and/or non-permissive to the entry of lymphocyte cells in the tumor area, comprising the quantification in a bioptic sample of said tumor of:
   - the expression of the hMENA11a marker in tumor cells and in the stromal cells of said sample;
   - the expression of the FN1 marker in the stroma of said sample
   - the expression of the CD3, CD8 and CD20 markers in the cells of said sample, considering both the central area of the tumor and its invasive margin; and
   - the expression of the PDL1 marker in the tumor cells of said sample
wherein,
the expression of hMENA11a higher than a cutoff value of 50, identified by ROC analysis, in the tumor cells of said sample; the absence of hMena11a expression in the stromal cells of said sample; the expression of the FN1 marker lower than an intensity value (also called score) equal to 2 in a staining intensity scale, from 0 to 3, in the stroma of said sample; the expression of the CD3, CD8 and CD20 markers in at least 5 cells per mm² in the tumor area or in the invasive margin of said sample; the presence of tertiary lymphoid structures (TLS) consisting of an aggregate of B lymphocytes within the structure and T cells outside of the same assessed respectively by the expression of the CD20 and CD3 markers, are representative of an immunogenic tumor microenvironment;
wherein:
   hMENA11a is the ENAH variant 1 ID: NM_001008493 splicing variant containing an additional exon (11a);
   hMENADv6, hMENA splicing variant lacking exon 6 ID: EU255274.1;
   FN1 is fibronectin 1 Gene ID: 2335
   PD-L1, is Programmed cell Death-ligand 1, alias CD274, Gene ID: 29126
while
the expression of hMena 11a lower than a cutoff value of 50 in the tumor cells of said sample; the expression of the FN1 marker equal to or greater than a cutoff value of score 2 in the stroma of said sample and the absence of expression of the CD3, CD8 and CD20 markers in cells within the tumor area or in the invasive margin of said sample are representative of an immunosuppressive tumor microenvironment;
a method for predicting the responsiveness or non-responsiveness, of patients with solid tumors, to treatment by immunotherapy optionally in combination with radiotherapy or chemotherapy or target therapy, which includes determination of the tumor microenvironment with the assay as described above and in any of the embodiments disclosed in the present description, wherein patients presenting an immunogenic tumor microenvironment will be responsive to immunotherapy treatment while patients presenting an immunosuppressive tumor microenvironment will be non-responsive to treatment by immunotherapy;
a method of therapeutic treatment comprising the above-described method, wherein patients for whom a responsiveness to immunotherapy is predicted are treated with immunotherapy, optionally in combination with radiotherapy or chemotherapy or target therapy, while patients for whom no responsiveness to immunotherapy is predicted will be treated with chemotherapy and/or radiotherapy and/or target therapy; also aimed at reconverting the hMena-positive stroma into a non-activated and permissive stroma (e.g., TGFbeta inhibitors -Fridman W 2017) and
a kit for the assessment of the microenvironment of a tumor, comprising:
   - reagents to quantify the expression of the hMENA11a marker in a biopsy sample;
   - reagents to quantify the expression of the FN1 marker in a biopsy sample;
   - reagents to quantify the expression of the CD3, CD8 and CD20 markers in a biopsy sample; and
   - reagents to quantify the expression of the PDL1 marker in a biopsy sample.

### DETAILED DESCRIPTION OF THE FIGURES

Figure 1. hMENA11a transfection significantly reduces extracellular matrix components in the secretoma of breast carcinoma cells, as demonstrated by mass spectrometry analysis. (a) Heat map of proteins secreted in different amounts in hMENAΔv6-expressing BT549 CNTR cells and hMENA11a -expressing clones (#14 and #104) (left). The analysis was conducted on three biological replicates. Quantification of the reduction of proteins associated with ECM composition is reported in the histograms (Right). (b) Heat map of proteins secreted differently between DAL CNTR cells and trasfectants stable for hMENA11a (left). The analysis was conducted on four biological replicates. Quantification of the reduction of proteins associated with ECM composition and also identified in BT549 cell line is reported in the histograms (Right). (c) Three networks identified by an algorithm of upstream regulators using Ingenuity Pathway Analysis (IPA).

In particular, the figure shows how the expression pattern of the two hMENA isoforms influences the composition of the extracellular matrix, which in turn plays a fundamental role in immune cell infiltration into the tumor, and therefore in response to therapy. Figure 2. hMENAΔv6 transfection increases, and hMENA11a transfection reduces fibronectin (FN1) expression of NSCLC cell lines. Low levels of FN1 in the stroma of tumors with a high expression of hMENA11a represent a favorable prognostic factor in early NSCLC patients. (a) Western blot of A549 cells transfected with empty vector (CNTR), hMENAΔv6 and hMENA11a with the indicated antibodies. The quantification of the increase or reduction of the expression of FN1 in cells transfected with hMENAΔv6 or hMENA11a is reported on the right (fold). (b) Consecutive sections of a representative NSCLC case labeled with Pan-hMENA, hMENA11a and FN1 antibodies. 20X enlargement. Scale Bar = 30 µm. (c) immunohistochemical characterization of 114 (lymph) node-negative NSCLCs, showing how the hMENA11a pos [high hMENA11a /low hMENA (t) as reported in Bria et al 2014] cases more frequently express low levels of FN1 in the stroma. (d-f) Kaplan-Meier evaluation of disease-free survival (DFS) of node-negative NSCLC patients according to dichotomized hybMENA11a and stromal FN1 expression.

The figure shows how the expression pattern of the two hMENA isoforms influences the expression of a fundamental component of the extracellular matrix which is fibronectin (FN1). Said data was obtained on cell lines and validated in tumor tissues, in which high levels of FN1 in the stroma surrounding the tumor are associated with low expression levels of hMENA11a in tumor cells. From a clinical standpoint, this association has a prognostic significance. Patients expressing high levels of hMENA11a in tumor cells and low levels of FN1 in the stroma have better prognosis. Figure 3. hMENA11a expression correlates with the presence of tertiary lymphoid structures (TLS) in the tumor area (AT) and low levels of stromal fibronectin in early (N0) NSCLC patients. Sections of a representative case decorated with antibodies toward CD3 (A), CD20 (B), hMENA11a (C) and Fibronectin (D). Magnification 100X. (E) Schematic TLSs model (F) Histograms represent the percentage of tumors categorized for the expression pattern of the hMENA isoforms (hMENA11a pos: hMENA11a high/Pan-hMENA low; hMENA11a neg: all other 3 cases) and show the presence (TLS pos) or absence (TLS neg) of TLS in the tumor area (AT). (G) Histograms represent the percentage of tumors categorized by low-level (FN low) or high-level (FN high) expression of fibronectin in the stroma; and show the presence (TLS pos) or absence (TLS neg) of AT TLSs. (H) Histograms represent the percentage of tumors categorized by the expression pattern of hMENA isoforms and fibronectin expression in the stroma (HybMENA11a pos /FN low; HybMENA11a neg /FN high;) and show the presence (TLS pos) or absence (TLS neg) of TLSs AT.

The figure shows how the expression of hMENA11a is associated to low expression levels of FN1 and to the presence of tertiary lymphoid structures in the tumor area. Therefore, the evaluation of hMENA isoforms expression, in parallel with the presence of T and B cells (lymphocytes) can add information related to the functional organization of the lymphocyte infiltrate.

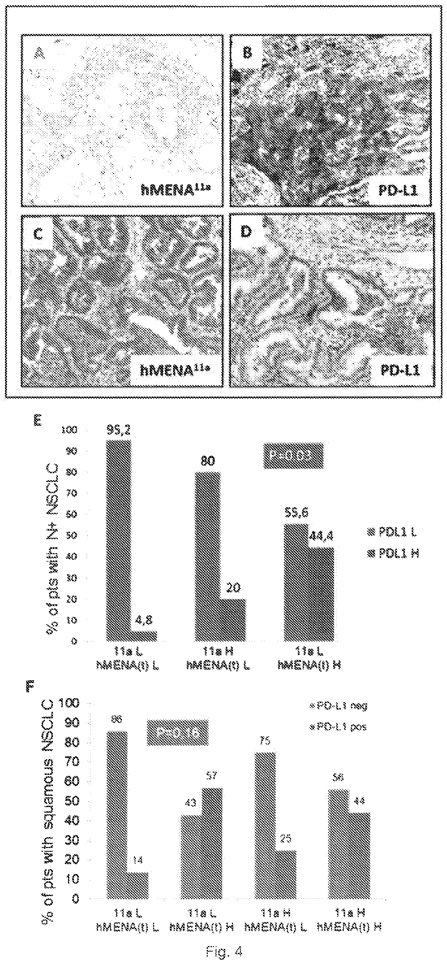

Figure 4. hMENA11a expression inversely correlates with PD-L1 expression in (lymph) node-positive (N+) NSCLC and in squamous NSCLC. Two representative cases. A-B, weak immunoreactivity with anti- hMENA11a (A) and strong positivity for PD-L1 (Dako, clone 22C3). C-D, high hMENA11a expression (C), and absence of PD-L1. Alveolar macrophages are positive for PD-L1. (E-F). Histograms show inverse correlation between the expression of hMENA11a and of PD-L1 in node-positive NSCLC (E) and in squamous NSCLC (F) patients.

In a case record of lung carcinoma patients, hMENA11a expression inversely correlates with PD-L1 expression.

Figure 5. Specific hMENA11a silencing induces increase of PD-L1 ligand at membrane protein level and of mRNA in lung carcinoma cells. EpT1Lu, H1650 e H1975 lung carcinoma cell lines were transfected with control siRNA (si-CNT), siRNAs specific for hMENA11a (si-11a) and siRNA for all hMENA isoforms (si-MENA). (A) PD-L1 levels analyzed by surface cytofluorimetric staining via specific monoclonal antibody. Bar graphs show percentage values of PD-L1 levels in EpT1Lu lines (left-side panel), H1650 (central panel) and H1975 (right-side panel). Each bar represents the mean percentage (± SEM) of 5 different experiments. P value calculated by t test. (B) Analysis by Western blotting of hMENA11a and Pan hMENA expressions by the related specific antibodies. Anti-tubulin antibody was used to normalize the amount of gel-loaded protein. (C) Analysis by Real-Time PCR of mRNA levels of PD-L1. Gene GAPDH was used as endogenous control.

The figure shows that hMENA11a silencing in various lung carcinoma cell lines induces an increase of PD-L1 protein expression on cells surface (A), and an increase of its messenger RNA (C), indicating that the assessment of hMENA isoforms expression can represent not only an indication of the composition of the extracellular matrix and the lymphocyte infiltrate, and therefore of a tumor microenvironment, immunogenic or immunosuppressive, but can be correlatable to an on-tumor expression of PD-L1, ligand of the main target of immunomodulatory therapies, indicating that a patient whose tumor expresses hMENA11a, even though PD-L1-negative, might be responsive to treatment. The invention suggests the need of a method including, besides the expression of PD-L1 - currently the sole marker used in clinical practice to select patients to be treated with inhibitors of this pathway - the expression of the above-cited microenvironment markers.

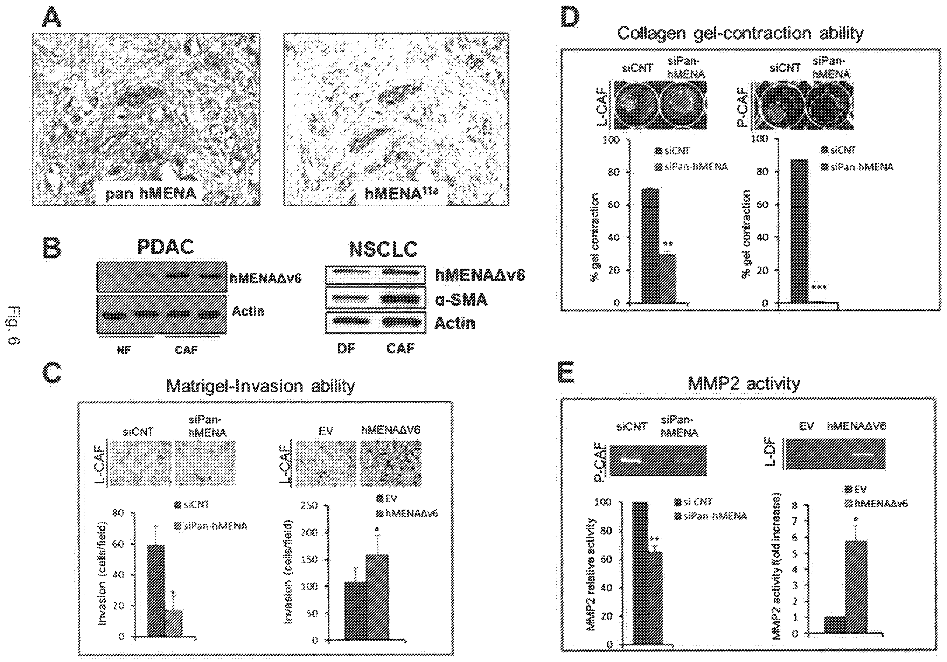

Figure 6. hMENA/hMENAΔv6, but not hMENA11a, are expressed in the stroma of pancreatic adenocarcinoma, and in particular in CAFs, where hMENAΔv6 modulates their functional activity. A. Immunohistochemical analysis, with pan hMENA and hMENA11a antibodies, of consecutive sections of a representative case of pancreatic adenocarcinoma. B. Western blots of lysates of normal fibroblasts (NF) and CAFs obtained from pancreatic tissue (PDAC) or from distal fibroblasts and CAFs obtained from NSCLC lung carcinoma with the indicated antibodies. C. Matrigel invention assay on CAFs obtained from lung adenocarcinoma (L-CAF) silenced for all hMENA isoforms (left) or transfected with hMENAΔv6 (right). D. Collagen gel contraction assay with CAFs obtained from lung (L-CAF) or pancreatic (P-CAF) adenocarcinoma, silenced for all hMENA isoforms. E. Zymogram for MMP2 activity performed with the conditioned medium of CAFs obtained from pancreatic adenocarcinoma (P-CAF), silenced for all hMENA isoforms (left), or of distal fibroblasts obtained from lung adenocarcinoma (L-DF), transfected with hMENAΔv6 (right).

The figure shows that hMENA/hMENAΔv6, and not hMENA 11a, is expressed in the stroma, and in particular in tumor-associated fibroblasts of lung and pancreatic tumors. In particular, the Δv6 isoform modulates the functional activity of tumor-associated fibroblasts, which in turn, by the production of extracellular matrix components and soluble factors, carry out an immunomodulating activity and therefore play a role in defining an immunosuppressive and/or non-permissive microenvironment. Said results suggest the need to introduce in the method of the invention the assessment of hMENA expression in the tumor stroma.

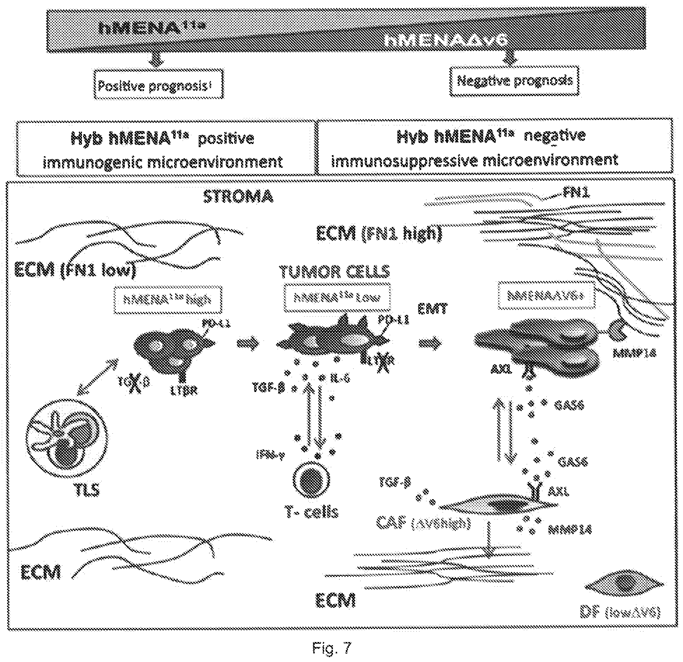

Figure 7. The figure shows a graphic summary of the transition from the expression of the "epithelial" and anti-invasive 11a isoform of hMENA to the expression of the "mesenchymal" proinvasive hMENADv6 isoform. Said transition influences signaling pathways important in the dialogue among tumor cell, ECM, CAFs and immune cells, causing a change of the tumor microenvironment from immunogenic to immunosuppressive. In-tumor detection of hMENA isoforms expression pattern in association with other markers, such as fibronectin (FN1) in the stroma, the presence/absence of lymphocyte infiltrate, also organized in tertiary lymphoid structures (TLS) in the tumor area, enables to identify the immunogenic or immunosuppressive characteristics of the tumor microenvironment, providing predictive information on patients' responsiveness to immunotherapy.

### GLOSSARY

For the purposes of the present description, the wording "tumor microenvironment" (TME) denotes, in accordance with the scientific literature, the environment in which the tumor develops and progresses, and is mainly comprised of extracellular matrix and cellular component, of which cancer-associated fibroblasts (CAFs), immune cells, among which T, B lymphocytes, tumor-associated macrophages, and endothelial cells and pericytes forming blood and lymph vessels are part. All these components crosstalk among them and with tumor cells, causing a highly dynamic situation.

The wording "immune checkpoints" in the present description denotes, in accordance with the scientific literature, proteins that control immune system activation, transmitting an inhibitory-type signal inside a T-lymphocyte (T-cell). Examples of these proteins include PD-1 and CTLA-4.

The wording "immune checkpoint inhibitors" in the present description denotes, in accordance with the scientific literature, inhibitory molecules tasked with eliminating restrictions to most of T-cell-mediated immune responses.

The wording "target therapy" in the present description denotes, in accordance with the scientific literature, a type of pharmacological therapy for the treatment of tumors addressed at opposing the specific mechanisms of the carcinogenesis process of the individual tumors, by interfering with molecules needed for tumor growth and progression. Drugs of this type are accordingly effective toward defined neoplasia types, and are therefore selective (hence, reference is also made to "customized therapy"). Target therapy intervenes on the mechanisms linked to oncogene and oncosuppressor gene expression, underlying the specific action of tumor promotion, entailing transformation of the cell from normal to pathological.

The wording "invasive margin" in the present description corresponds to that commonly accepted in oncologic histology and denotes the region of the histological section at the boundary between tumor cells and untransformed tissue. In the literature, by invasive margin it is meant a region of about 500 micrometers on each side of the boundary between tumor tissue and untransformed tissue cells.

The wording "tertiary lymphoid structures" (TLS) in the present description denotes, in accordance with the scientific literature, lymph node-like structures present in many solid tumors, comprised of T cells and mature dendritic cells surrounded by a B lymphocyte follicle.

For the purposes of the present invention
- hMENA alias ENAH, Gene ID: 55740
- hMENA11a, Enah variant 1 ID: NM_001008493 splicing variant containing an additional exon (11a)
- hMENADv6, hMENA splicing variant lacking exon 6 ID: EU255274.1
- hMENA11a: HybMENA11a hybrid variable pos= [high hMENA11a /low hMENA (t)]; HybMENA11a neg (all other 3 cases)
- FN1 fibronectin 1 Gene ID: 2335
- PD-L1, alias CD274, Gene ID: 29126
- CD3, T cell co-receptor, consisting of one CD3γ chain, one CD3δ chain, and two CD3ε chains
- CD20 B lymphocyte marker, Gene ID: 931
- CD4, Gene ID: 920
- CD8, Gene ID: 925

In the present description, the terms total hMena or pan-hMena are used as synonyms.

### DETAILED DESCRIPTION OF THE INVENTION

The present invention therefore enables to define the tumor microenvironment of solid tumors and to predict responsiveness of patients affected by said tumors to immunotherapy on the basis of said microenvironment.

Therefore, object of the invention is an assay to assess the microenvironment, immunogenic or immunosuppressive, of a tumor comprising the quantification in a bioptic sample of said tumor:
- the expression of the hMena11a marker in tumor cells and in the stromal cells of said sample;
- the expression of the FN1 marker in the stroma of said sample;
- the expression of the CD3, CD8 and CD20 markers in the cells of said sample; and
- the expression of the PDL1 marker in the tumor cells of said sample
wherein,
the expression of hMena11a higher than a cutoff value of 50, identified by ROC analysis, in the tumor cells of said sample; the absence of hMena11a expression in the stromal cells of said sample; the expression of the FN1 marker lower than an intensity value (score) 2 in a staining intensity scale, from 0 to 3 in the stroma of said sample; the expression of the CD3, CD8 and CD20 markers in at least 5 cells per mm² in the tumor area or in the invasive margin of said sample; the presence of tertiary lymphoid structures (TLS) consisting of an aggregate of B lymphocytes within the structure and T cells outside of the same, assessed respectively by the expression of markers CD20 and CD3, are representative of an immunogenic tumor microenvironment;
wherein:
   hMENA11a is the ENAH variant 1 ID: NM_001008493 splicing variant containing an additional exon (11a);
   hMENADv6, hMENA splicing variant lacking exon 6 ID: EU255274.1;
   FN1 is fibronectin 1 Gene ID: 2335
   PD-L1, is Programmed cell Death-ligand 1, alias CD274, Gene ID: 29126;
   while the expression of hMena11a lower than a cutoff value of 50 in the tumor cells of said sample; the expression of the FN1 marker equal to or greater than a cutoff value of score 2 in the stroma of said sample and the absence of expression of the CD3, CD8 and CD20 markers in cells within the tumor area or in the invasive margin of said sample are representative of an immunosuppressive tumor microenvironment.

The presence/absence of TLSs (tertiary lymphoid structures) can be assessed by morphological analysis of the immunohistochemical staining of CD3, CD8 and CD20, in which clusters of CD20+ cells surrounded by CD3-positive areas are considered TLSs. In one embodiment of the above-described assay, the expression in tumor and stromal cells of said sample of the hMena ΔV6 marker and/or the total hMena marker expression can be further quantified, wherein said total hMena marker comprises all hMena protein isoforms, and wherein:
the absence of hMena ΔV6 expression in tumor cells of said sample and/or the total hMena expression lower than a cutoff value di 80 in the tumor cells of said sample; and the absence of any of said markers in the stroma of said sample are representative of an immunogenic tumor microenvironment; while
the presence of hMena ΔV6 expression in tumor cells of said sample and an expression thereof in the stromal cells of said sample and/or
the presence of total hMena expression greater than a cutoff value of 80 in the tumor cells and an expression thereof in the stromal cells of said sample are representative of an immunosuppressive tumor microenvironment.

The quantifications in the above-described assay can be performed, for instance, by immunohistochemistry methods as explained in more detail hereinafter. The detection of the two different hMENA isoforms can therefore be performed with specific antibodies for each isoform, i.e. antibodies specifically binding only a single hMena isoform. In case of antibodies specific for the 11a isoform, and therefore binding exclusively said isoform, the antibodies can bind, e.g., epitopes present in exon 11a, and therefore absent in all other isoforms.

In case of antibodies specific for the Δv6 isoform, and therefore binding exclusively said isoform, the antibodies can bind epitopes astride exon 6 deletion sites, therefore absent in all other isoforms.

Antibodies specific for hMENA isoforms are described in the literature.

Antibodies specific for total hMena, i.e. antibodies binding specifically epitopes common to all hMENA isoforms, and therefore binding exclusively hMena protein in all his forms, are also described in the literature.

In the figures appended to the present description, data of experiments performed with antibodies specific for the 11a isoform of hMena and with antibodies specific for all hMENA isoforms (also called anti-total hMena, o pan-hMena antibodies) are depicted. In this case, the data obtained with these two markers are also representative of the Δv6 isoform (as confirmed by analyses performed on mRNAs).

For instance, the staining for pan-hMENA and hMENA11a can be quantified both by an intensity value (score) from 0 to 3 and by the percentage of positive cells. A continuous variable is generated as product of intensity and percentage, obtaining a numerical value from 0 to 300. Then, a ROC analysis is performed in order to find the optimal cutoff of the two variables. The identified cutoff is different for the two variables (80 for pan-hMENA and 50 for hMENA11a).

By pan-hMENA low expression (low), for the purposes of the present invention a value of <80 is meant, whereas by pan-hMENA high expression (high) a value of >80 is meant. As to hMena11a, by low expression (low), for the purposes of the present invention a value of <50 is meant, whereas by hMENA11a high expression (high) a value of >50 is meant. Therefore, for equal values of pan-hMENA and hMENA11a (e.g., 60) pan-hMENA low (as <80) and hMENA11a high (as >50) will be had. As indicated above, the expression value is calculated as product between staining intensity (score 0, 1, 2, 3) and percentage of positive cells (0-100), whereas the optimal cutoff value for the two variables has been identified by a ROC analysis (Bria 2014, above). It is known that in tumor cell lines the expression of hMENA11a and hMENADv6 is alternative, and positivity for pan-hMENA is always accompanied by positivity for one or the other of the hMENA11a and hMENADv6 isoforms. These data were obtained by western blot, using antibodies specific for hMENA11a; hMENADv6 and pan-hMENA, which recognizes all hMENA isoforms. On the basis of these preclinical results on cell line models, it can be speculated that the group of tumors that are pan-hMENA high/hMENA11a low includes tumors expressing the hMENAΔv6 isoform, even though this is not directly assessable owing to the lack of an antibody specific for hMENAΔv6 that works in immunohistochemistry.

The computation of FN1 marker expression is performed by using an extremely simple staining intensity scale, from 0 to 3: score 0: no staining; score 1: weak, 2: moderate, and 3: strong. Scores 0-1 are merged and labeled as 'Low', whereas scores 2-3 are combined and labeled as 'High'. A person skilled in the field could evaluate staining intensity with respect to other markers used in histopathological practice as positive controls, indicating the occurred staining reaction; such controls, as is known, vary tumorwise and a person skilled in the field will be able to select from the literature the most suitable markers. As mentioned above, in one of the embodiments of the invention, the assay according to any one of the above-described embodiments, the expression of the various markers is quantified by immunohistochemistry techniques with specific antibodies that selectively bind each marker, i.e.: specific antibodies that selectively bind the 11a isoform of hMENA of the hMena protein, specific antibodies that selectively bind CD3, specific antibodies that selectively bind CD8, specific antibodies that selectively bind CD20 and specific antibodies that selectively bind FN1 and, optionally, with specific antibodies that selectively bind the hMena ΔV6 isoform of the hMena protein and/or anti-total hMena antibodies, i.e. antibodies that bind to the hMena protein in all its known isoforms. Hence, specific for the hMena protein, but unable to recognize the isoforms. One example of said antibodies are antibodies targeting epitopes that are not in exon 11a and that are not included in the zone in which exon 5 is joined to exon 7, typical of the deltaV6 form.

The immunohistochemistry techniques can be performed according to any method known to the technician in the field, on bioptic samples that are fresh, frozen or stored in paraffin, also by including (embedding) therein.

For the purposes of the present invention, suitable bioptic samples can be samples obtained by standard techniques known to the technician in the field, such as biopsy, surgery or suction.

For instance, the expression can be quantified on consecutive sections of the bioptic sample by using one section for each marker, and then using standard immunohistochemistry techniques with secondary antibodies labeled with peroxidase-conjugated streptavidin, or alternatively by using polymers with the Polymer Refine Detection Bond method.

According to an alternative embodiment, the expression of the markers indicated in any one of the embodiments described herein, immunohistochemistry techniques can be used that envisage the use of secondary antibodies labeled with different fluorochromes and the detection of the different markers, also on the same section of the bioptic sample, such as, e.g., by sequential or simultaneous multiplex immunohistochemistry under fluorescence (fICH) as described in the literature. Depending on the preferred detection system, the person skilled in the field will use primary antibodies as described, specially provided secondary antibodies and suitable image acquisition devices. In any case, for quantification by immunohistochemistry the technician in the field could use any standardized immunohistochemistry technique to perform the assay of the invention.

Alternatively, the quantification as described herein can be performed by detecting the mRNA of each of said markers, e.g., by QRT-PCR or RNA-Seq techniques.

On the basis of the assay described herein, object of the invention is also a method for predicting the responsiveness or non-responsiveness, of patients with solid tumors, to treatment by immunotherapy, optionally in combination with radiotherapy or chemotherapy or target therapy, which includes assessment of the tumor microenvironment with the assay as described in any one of the embodiments indicated in the present description, wherein patients presenting an immunogenic tumor microenvironment will be responsive to immunotherapy treatment, while patients presenting an immunosuppressive tumor microenvironment will be non-responsive to treatment by immunotherapy.

What described for any embodiment of the assay of the invention applies to the method for predicting the responsiveness to treatment by immunotherapy and to the therapeutic method providing a step of classifying the patient to be treated as responsive or non-responsive to immunotherapy.

Therefore, object of the invention is also a therapeutic method for the treatment of patients with solid tumors, wherein, prior to defining the therapeutic regimen, the patient is subjected to a method of classification as responsive or non-responsive to immunotherapy as described above, and wherein, if the patient is classified as responsive, immunotherapy is administered, optionally combined with radiotherapy, chemotherapy, or target therapy, while, if the patient is classified as non-responsive, chemotherapy, or radiotherapy, or a target therapy is administered, without administration of immunotherapic drugs or active ingredients.

According to the present description, immunotherapy can be carried out with any immunotherapic drug for solid tumors commonly used in therapy.

A non-limiting example of such drugs comprises therapeutic vaccines or immune (immunological) checkpoint inhibitors.

In one embodiment, immunotherapy can be carried out with one or more immune checkpoint inhibitor drugs as defined in the glossary. A non-limiting example of immune checkpoint inhibitor drugs is represented, e.g., by Ipilibumab, Nivolumab or Pembrolizumab.

Ipilimumab (Yervoy) is a monoclonal IgG1 antibody binding CTLA-4 and preventing the reduction of cytotoxic T cells.

PD-1 signaling pathway blockage is deemed to modulate the activity of T cells that have become non-responsive after contacting PD-1 ligands in the tumor microenvironment.

Currently, there are two anti-PD-1 drugs at an advanced development stage: Nivolumab and Pembrolizumab.
a) Nivolumab (BMS-936558), a completely human immunotherapic drug, IgG4 inhibitor of immune checkpoint PD-1
b) Pembrolizumab (MK-3475) is a humanized IgG4 monoclonal antibody binding PD-1. Anti-PD-L1: anti-PD-L1 therapies also target the PD-1 signaling pathway by binding with one of its ligands, e.g., PD-L1.

As mentioned above, immunotherapy could be administered in combination (simultaneous or sequential) with radiotherapy, chemotherapy or target therapy. Examples of target therapy drugs may be given from drugs of humanized monoclonal antibody category of humanized monoclonal antibodies (trastuzumab, cetuximab), which act by interfering with the receptors present on the cell surface, as in the case of the epidermal growth factor receptor (EGFR), one of the target molecules proved more effective for target therapy. Another example are Small molecules (Erlotinib, Imatinib) which penetrate the cell membrane to interact with the target protein in the cell, interfering with the enzymatic activity of the protein.

A further object of the invention is a kit for the assessment of the microenvironment of a tumor, comprising:
- reagents to quantify the expression of the hMena 11a marker in a biopsy sample;
- reagents to quantify the expression of the FN1 marker in a biopsy sample;
- reagents to quantify the expression of the CD3, CD8 and CD20 markers in a biopsy sample; and
- reagents to quantify the expression of the PDL1 marker in a biopsy sample.

According to a further embodiment, the kit can further comprise reagents to quantify the expression of the hMena ΔV6 marker and/or reagents to quantify the expression of the total hMena marker, i.e. of all the hMena protein isoforms in a biopsy sample.

The kit of the invention can be a kit for detection by immunohistochemistry, or for detection by nucleic acids analysis, or both.

In the case of immunohistochemical kits, the reagents for detection can comprise specific antibodies that selectively bind the 11a isoform of hMENA of the hMena protein, specific antibodies that selectively bind CD3, specific antibodies that selectively bind CD8, specific antibodies that selectively bind CD20 and specific antibodies that selectively bind FN1 and, optionally, specific antibodies that selectively bind the hMena ΔV6 isoform, and/or anti total hMena antibodies specifically binding all Hmena protein isoforms.

The kit can further comprise secondary antibodies labeled, e.g., with peroxidase-conjugated streptavidin, or labeled with a different fluorochrome for each secondary antibody depending on the desired immunohistochemical method as described above, or the detection can comprise the Polymer Refine Detection Bond system. Alternatively, or jointly, the kit according to any embodiment described herein can comprise as reagents oligonucleotides specific for detecting mRNA of each of said markers.

The sequences of the selected markers are known in the literature (as reported above) and primers specifically amplifying said sequences are also known. For instance, as is of common knowledge, for hMena variants primer sequences selectively amplifying isoforms are used, for hMena11a primers are used which specifically amplify regions comprising in whole or in part exon 11a, for the commonly known hMena isoform primer sequences are used which amplify one or more regions comprising the whole exon 11 or part thereof, as well as the whole exon 6 or part thereof, for the hMena ΔV6 isoform, instead, primers comprising the zone astride exon 5 and exon 7 are used. Hereinafter, examples of embodiments of the invention are provided. These examples are not aimed at limiting the invention but are merely to provide possible embodiments as a guide for the technician in the field.

The use of conventional techniques different from those described below is envisaged among the embodiments of the invention.

The following examples aim at illustrating ways for carrying out the invention but are not intended to limit the protection scope thereof.

All analyses performed on bioptic samples reported herein were performed on samples collected from informed patients who gave their consent to the treatment of said samples.

### EXAMPLES

### 1. Immunohistochemical analysis

Tissues fixed 18-24 h in 4% formaldehyde are included in paraffin. Immunohistochemical labeling is performed on tissues of about 5-µm thickness. Sections are mounted on SuperFrost Plus slides (Menzel-Glaser, Braunschweig, Germany) and, once deparaffined and rehydrated, are treated in a thermostated bath at 96°C for 40 minutes in a 10 mmol/L citrate solution (pH 6). Immunohistochemical labeling for hMENA11a; pan-hMENA; CD3, CD20; CD4; CD8; PD-L1 is preferentially detected by Bond Polymer Refine Detection (Leica Biosystem, Milan, Italy) with an automatic autostainer (BondTM Max, Leica). Reactivity toward hMENA isoforms by immunohistochemistry is assessed with 4 different scores: no staining, score 0; weak diffused staining in <10% of cells, score 1+; moderate cytoplasmatic staining in 10% - 70% of cells, score 2+; intense staining in >70% of cells; in the presence of membrane reinforcement, score 3+.

Fibronectin expression in the stroma is assessed by using a scale from 0 to 3: score 0: no staining; score 1: weak, 2: moderate, and 3: intense). Grades 0-1 are merged and labeled as 'Low', whereas grades 2-3 are combined and labeled as 'High'.

PD-L1 expression is considered positive if at least 50% of tumor cells are positive.

The expression study of CD3, CD20 CD4, CD8 markers is assessed by dividing the tissues into two areas: invasive margin and tumor central area. The expression of CD3, CD20 CD4, CD8 markers is assessed by immunohistochemistry, counting the number of positive cells per mm² in the tumor central area and its invasive margin.

Cell count per mm² and the relative computation can be performed by digital pathology with a dedicated software, and the result is given by the combination of the 4 markers for the two regions analyzed.

Alternatively, the expression of CD3, CD20 CD4, CD8 markers in tumor tissues can also be assessed at mRNA level by QRT-PCR, using primers or probes known to a technician in the field, or by RNA-Seq.

The presence/absence of TLS is assessed by morphological analysis of the immunohistochemical staining of CD3 and CD20, in which CD20+ cell clusters surrounded by CD3-positive areas are considered TLSs.

### Experimental results

### 1. hMENA^{11a} modifies ECM composition.

A proteomic analysis was performed by mass spectrometry on the secretoma of two clones of breast carcinoma cells BT549 transfected for 11a isoform of hMENA and on control BT549 cells not expressing hMENA^{11a} but expressing the hMENADv6 isoform. Among 1719 proteins identified in the conditioned medium (CM) of the cells, 373 proteins (10% false discovery rate (FDR), q = 0.1) differently expressed in the two hMENA^{11a} clones with respect to control cells were observed (Figure 1a). Upstream regulator analysis, performed with Ingenuity Pathway (Qiagen, MD) had identified the downregulation of different pathways important in BT549/hMENA^{11a} clones. TGFβ1 activation pathway demonstrated to be the most significant (Activation z-score = - 4,389, p-value = 1,12E-20), along with the FN1 pathway (Activation z-score = -2.472 p-value = 1.79E-06) and β1 integrin (Activation z-score = -2.182, p-value = 0.000282) (Figure 1c).

Said data were confirmed in another breast carcinoma cell line, DAL, which expresses undetectable levels of hMENA(t), stably transfected with hMENA^{11a}. Secretoma analysis showed that 1324 proteins were significantly different in the CM of cells transfected for hMENA^{11a} and in the control cells (Figure 1b). Among differentlyexpressed proteins, ECM and metalloproteinase component reduction were observed in both BT549 hMENA^{11a} and DAL hMENA^{11a}, compared with control cells (Figure 1a, b).

### 2. hMENA^{11a} "high" expression, along with "low" stromal FN1 prolongs disease-free survival in node-negative NSCLC patients.

Considering the mass spectrometry results showing how a high hMENA^{11a} /hMENAΔv6 expression ratio decreases the secretion of a cluster of proteins belonging to the FN1 pathway and considering the relevance of FN1 expression in lung carcinoma, the Inventors evaluated the effect of hMENA^{11a} and hMENAΔv6 transfection on FN1 expression of lung carcinoma lines. The results have shown that hMENA^{11a} transfection reduces, whereas hMENAΔv6 increases FN1 level (Figure 2a, b).

To confirm the clinical relevance of these experimental results, the Inventors analyzed FN1 the expression in 114 tumor tissues from early node-negative NSCLC patients, in parallel with Pan-hMENA and hMENA^{11a} staining, assessed as hybMENA^{11a} - positive (comprising hMENA^{11a} high / hMENA(t) low cases) or -negative (comprising all other cases, and therefore hMENAΔv6-expressing tumors). The results show that FN1 expression in the stroma is differently distributed in hybMENA^{11a} -positive or -negative tumors (Figure 2c, d) (p = 0.03). High FN1 was expressed more significantly in the hybMENA^{11a}-negative group, including hMENAΔv6-expressing tumors. On the contrary, hybMENA^{11a}-positive tumors fundamentally express low FN1 levels in the stroma, in accordance with proteomics data obtained in the cell lines. Of clinical relevance, Kaplan-Meier curves for DFS, in accordance with the combination of the two variables, indicate that 72.5% of patients with hybMENA^{11a}-positive and low FN1 are disease-free at 5 years (p < 0.0001), whereas, when considering only hybMENA^{11a} expression, the Inventors found that 63.5% of patients is disease-free at 5 years (Figure 2e). These data indicate that the combination of high hMENA^{11a} and low stromal FN1 represents a promising prognostic factor in early node-negative NSCLC patients.

### 3. hMENA^{11a} expression correlates with tertiary lymphoid structures (TLS) presence and low fibronectin levels in tumor tissues of early node-negative NSCLC patients

The research group of the Authors of the present invention reported that hMENA^{11a} expression correlates with a favorable prognosis in a group of NSCLC patients (Bria, 2014). An accurate immunohistochemical analysis for the presence of TLS in 192 cases of early (node-negative) NSCLC showed the presence of TLS in the tumor area (assessed as clusters of CD20+ cells surrounded by CD3+ cells) in 46 cases on 158 examined. 48 (25%) cases exhibited reactivity for hMENA^{11a}. TLS presence in the tumor area is moreover significantly associated with high positivity for hMENA^{11a} (hMENA^{11a} positive p<0.0001) (Figure 3f).

Since mass spectrometry data obtained by the research group of the Inventors indicate that hMENA expression pattern influences ECM composition, which in turn can influence immune cell infiltration, the Inventors assessed fibronectin expression in the stroma of the analyzed cases, finding that tumors with high FN1 in the stroma have less TLS (Figure 3g) and that hybMENA^{11a}-positive tumors exhibit low levels of FN1 in the stroma, and more frequently TLS in the tumor area (p=0.003) (Figure 3h).

### 4. hMENA^{11a} expression inversely correlates with PD-L1 expression in 40 node-positive (N+) NSCLCs and in 45 squamous NSCLCs. hMENA^{11a} specific silencing induces increase of PD-L1 ligand at membrane protein level, and of mRNA in lung carcinoma cells.

An immunohistochemical analysis for the expression of PD-L1 (ligand of immune checkpoint PD-1) on a case record of 40 positive-node NSCLC cases in parallel with the assessment of the expression of hMENA(t) and hMENA^{11a} demonstrated that only 20% of tumors expressing "high" hMENA^{11a} and "low" hMENA(t) express PD-L1, which is instead expressed in 44.4% of "low" hMENA^{11a} and "high" hMENA(t) cases (Figure 4e). Similar results were obtained in a case record of 45 cases of squamous NSCLCs (Figure 4f). Considering said data, the Inventors assessed, in experimental models of lung carcinoma cell lines, whether hMENA^{11a} expression might influence PD-L1 expression. The results obtained show that hMENA^{11a} specific silencing by interfering RNA (siRNA) induces an increase of PD-L1 levels both at a cell membrane protein level, as evaluated by cytofluorimetry (Figure 5a), and at RNA level, as assessed by QRT-PCR (Figure 5c). On the contrary, the silencing of all hMENA isoforms by a siRNA that complexes to an RNA sequence common to all hMENA transcripts does not influence PD-L1 expression. These data indicate that hMENA^{11a} expression level in tumor cells can influence T lymphocyte phenotype.

### 5. hMENA/hMENAΔv6, but not hMENA^{11a}, are expressed in pancreatic carcinoma stroma, and in particular in CAFs where hMENADv6 modulates their functional activity

The research group of the Inventors published that the expression patterns of hMENA isoforms represents a prognostic factor in pancreatic carcinoma, one of the tumors more resistant to immune checkpoint inhibitors, with hMENA^{11a} expression associated with a favorable prognosis (Mechionna 2016). An accurate immunohistochemical analysis shows that hMENA/hMENADv6, and not hMENA^{11a} is expressed in the stroma and in particular in tumor-associated fibroblasts (Figure 6A). By using primary cultures of fibroblasts obtained from surgical collections of pancreatic tumors (CAFs) or of normal pancreatic islets (NFs) and of fibroblasts obtained from the tumor area (CAFs) or the distal area (DFs) of surgical collections of NSCLC lung cancers, the Inventors demonstrated that CAFs express higher hMENADv6 levels compared to NFs or DFs (Figure 6B). Functional studies further demonstrated that hMENADv6 isoform modulates CAF functional activity. In fact, hMENADv6 silencing or overexpression respectively reduces or increases CAF ability to invade a matrigel matrix (Figure 6C) and to produce active metalloproteinase 2 (MMP2) (Figure 6E). Moreover, hMENA/hMENADv6 silencing reduces a characteristic CAF function, which is the ability to contract collagen gel (Figure 6D). Said results suggest the need to introduce in the method of the invention the assessment of the expression of hMENA in the tumor stroma.

## Claims

1. An assay to assess the microenvironment, immunogenic or immunosuppressive, of a tumor comprising the quantification in a bioptic sample of said tumor of:
- the expression of the hMENA11a marker in tumor cells and in the stromal cells of said sample;
- the expression of the FN1 marker in the stroma of said sample;
- the expression of the CD3, CD8 and CD20 markers in the cells of said sample; and
- the expression of the PDL1 marker in the tumor cells of said sample
wherein,
the expression of hMENA11a higher than a cutoff value of 50, identified by ROC analysis, in the tumor cells of said sample; the absence of expression of hMENA11a in the stromal cells of said sample; the expression of the FN1 marker lower than an intensity value, called score, equal to 2 in a staining intensity scale, from 0 to 3 in the stroma of said sample and the expression of the CD3, CD8 and CD20 markers in at least 5 cells per mm² in the tumor area or in the invasive margin of said sample; the presence of tertiary lymphoid structures (TLS) consisting of an aggregate of B lymphocytes within the structure and T cells outside of the same, assessed respectively by the expression of CD20, CD8 and CD3 markers, are representative of an immunogenic tumor microenvironment, while
the expression of hMENA1 1a lower than a cutoff value of 50 in the tumor cells of said sample; the expression of the FN1 marker equal to or greater than a cutoff value of score 2 in the stroma of said sample and the absence of expression of the CD3, CD8 and CD20 markers in cells within the tumor area or in the invasive margin of said sample are representative of an immunosuppressive tumor microenvironment;
wherein:
hMENA11a is the ENAH variant 1 ID: NM_001008493 splicing variant containing an additional exon (11a);
hMENADv6, hMENA splicing variant lacking exon 6 ID: EU255274.1;
FN1 is fibronectin 1 Gene ID: 2335
PD-L1, is Programmed cell Death-ligand 1, alias CD274, Gene ID: 29126.

2. The assay according to claim 1, wherein the expression of the marker hMena ΔV6 and/or of the marker total hMena, wherein said total hMena marker comprises all the hMena protein isoforms, is further quantified in the tumor and stromal cells of said sample, wherein:
the absence of hMena ΔV6 expression in tumor cells of said sample and/or the total hMena expression lower than a cutoff value of 80 in the tumor cells of said sample; and the absence of any of said markers in the stroma of said sample are representative of an immunogenic tumor microenvironment; while the presence of hMena ΔV6 expression in tumor cells of said sample and an expression thereof in the stromal cells of said sample and/or
the presence of total hMena expression greater than a cutoff value of 80 in the tumor cells and an expression thereof in the stromal cells of said sample are representative of an immunosuppressive tumor microenvironment;

3. The assay according to any of claims 1 or 2, wherein said expression is quantified by immunohistochemical techniques with specific antibodies that selectively bind the 11a isoform of hMENA of the hMena protein, specific antibodies that selectively bind CD3, specific antibodies that selectively bind CD8, specific antibodies that selectively bind CD20 and specific antibodies that selectively bind FN1 and, optionally, specific antibodies that selectively bind hMena ΔV6 isoform of hMena protein and/or anti-total hMena-specific antibodies, i.e. antibodies that bind all isoforms of the hMena protein.

4. The assay according to claim 3, wherein said expression is quantified on consecutive sections of said sample, each for each marker, with detection techniques using secondary antibodies labeled with peroxidase-conjugated streptavidin or alternatively using dextran polymers conjugated to peroxidase or directly to dyes.

5. The assay according to claim 4, wherein said expression is quantified on one or more sections of said sample, with detection techniques using secondary antibodies labeled with a different fluorochrome for each secondary antibody.

6. The assay according to any of claims 1 or 2, wherein said expression is quantified by detecting mRNA of each of said markers.

7. The assay according to any of claims 1 to 6, wherein said biopsy sample is a sample included in paraffin.

8. A method for predicting the responsiveness or non-responsiveness, of patients with solid tumors, to treatment by immunotherapy optionally in combination with radiotherapy or chemotherapy or target therapy, which includes determination of the tumor microenvironment with the assay as described in any of the claims 1 to 7, wherein patients presenting an immunogenic tumor microenvironment will be responsive to immunotherapy treatment while patients presenting an immunosuppressive tumor microenvironment will be non-responsive to treatment by immunotherapy.

9. The method according to claim 8, wherein said immunotherapy is carried out with one or more immunological checkpoint inhibitor drugs.

10. A kit for the assessment of the microenvironment of a tumor, comprising:
- reagents to quantify the expression of the hMENA11a marker in a biopsy sample;
- reagents to quantify the expression of the FN1 marker in a biopsy sample;
- reagents to quantify the expression of the CD3, CD8 and CD20 markers in a biopsy sample; and
- reagents to quantify the expression of the PDL1 marker in a biopsy sample.

11. A kit according to claim 10, further comprising reagents for quantifying the expression of the hMena ΔV6 marker and/or reagents to quantify the expression of the total hMena marker, i.e. of all the hMena protein isoforms in a biopsy sample.

12. Kit according to any of claims 10 or 11, wherein said reagents comprise specific antibodies that selectively bind the 11a isoform of hMENA of the hMena protein, specific antibodies that selectively bind CD3, specific antibodies that selectively bind CD8, specific antibodies that selectively bind CD20 and specific antibodies that selectively bind FN1 and, optionally, with specific antibodies that selectively bind the hMena ΔV6 isoform of hMena protein and/or anti-total hMena-specific antibodies, i.e. antibodies that bind all isoforms of the hMena protein.

13. The kit according to claim 12, further comprising secondary antibodies labeled with peroxidase-conjugated streptavidin or alternatively comprising dextran polymers conjugated to peroxidase or directly to dyes.

14. The kit according to claim 12, further comprising secondary antibodies labeled with a different fluorochrome for each secondary antibody.

15. The kit according to any of claims 10 or 11, wherein said reagents are oligonucleotides specific for the detection of the mRNA of each of said markers.

## Patentansprüche

1. Prüfung zum Bewerten der immunogenen oder immunsuppressiven Tumormikroumgebung umfassend die Quantifizierung in einer bioptischen Probe des Tumors der:
- Expression des hMENA11a-Markers in Tumorzellen und in Stromazellen der Probe;
- Expression des FN1-Markers in dem Stroma der Probe;
- Expression der CD3-, CD8- und CD20-Marker in den Zellen der Probe; und
- der Expression des PDL1-Markers in den Tumorzellen der Probe,
wobei,
die Expression von hMENA11a in den Tumorzellen der Probe höher als ein durch ROC-Analyse ermittelter Cutoff-Wert von 50; die Abwesenheit der Expression von hMENA11a in den Stromazellen der Probe; die Expression des FN1-Markers im Stroma der Probe niedriger als ein Intensitätswert, Score genannt, gleich 2 in einer Färbeintensitätsskala von 0 bis 3, und die Expression der CD3-, CD8- und CD20-Marker in mindestens 5 Zellen pro mm² im Tumorbereich oder im invasiven Rand der Probe; das Vorhandensein von tertiären lymphoiden Strukturen (TLS) bestehend aus einem Aggregat von B-Lymphozyten innerhalb der Struktur und T-Zellen außerhalb derselben, bewertet jeweils durch die Expression der CD20-, CD8- und CD3-Marker, repräsentativ sind für eine immunogene Tumormikroumgebung, während die Expression von hMENA11a kleiner als ein Cutoff-Wert von 50 in den Tumorzellen der Probe; die Expression des FN1-Markers gleich oder größer als ein Cutoff-Wert von Score 2 in dem Stroma der Probe und die Abwesenheit der Expression der CD3-, CD8- und CD20-Marker in Zellen innerhalb des Tumorbereichs oder in dem invasiven Rand der Probe repräsentativ für eine immunsuppressive Tumormikroumgebung sind;
wobei:
hMENA11a die ENAH-Variante 1 ID: NM_001008493 Spleißvariante ist, die ein zusätzliches Exon (11a) enthält;
hMENADv6, hMENA-Spleißvariante, der das Exon 6 ID: EU255274.1 fehlt;
FN1 Fibronektin 1 Gen ID: 2335 ist;
PD-L1, Programmed cell Death-ligand 1, alias CD274, Gen ID: 29126 ist.

2. Prüfung gemäß Anspruch 1, wobei die Expression des Markers hMena ΔV6 und/oder des Markers Gesamt-hMena, wobei der Gesamt-hMena-Marker alle hMena-Proteinisoformen umfasst, in den Tumor- und Stromazellen der Probe weiter quantifiziert wird, wobei:
die Abwesenheit der hMena ΔV6-Expression in den Tumorzellen der Probe und/oder die Gesamt-hMena-Expression unter einem Cutoff-Wert von 80 in den Tumorzellen der Probe; und die Abwesenheit jeglicher dieser Marker in dem Stroma der Probe repräsentativ für eine immunogene Tumormikroumgebung sind; während
das Vorhandensein einer hMena ΔV6-Expression in Tumorzellen der Probe und einer Expression davon in den Stromazellen der Probe und/oder
das Vorhandensein einer hMena-Gesamtexpression größer als ein Cutoff-Wert von 80 in den Tumorzellen und eine Expression davon in den Stromazellen der Probe repräsentativ für eine immunsuppressive Tumormikroumgebung sind;

3. Prüfung gemäß einem der Ansprüche 1 oder 2, wobei die Expression durch immunhistochemische Techniken mit spezifischen Antikörpern quantifiziert wird, die selektiv die 11a-lsoform von hMENA des hMena-Proteins binden, mit spezifischen Antikörpern, die selektiv CD3 binden, mit spezifischen Antikörpern, die selektiv CD8 binden, mit spezifischen Antikörpern, die selektiv CD20 binden, und mit spezifischen Antikörpern, die selektiv FN1 binden, und optional mit spezifischen Antikörpern, die selektiv hMena ΔV6-Isoform des hMena-Proteins binden, und/oder mit antitotalen hMenaspezifischen Antikörpern, das heißt Antikörpern, die alle Isoformen des hMena-Proteins binden.

4. Prüfung gemäß Anspruch 3, wobei die Expression an aufeinanderfolgenden Schnitten der Probe, jeweils für jeden Marker, mit Nachweistechniken unter Verwendung von mit Peroxidase-konjugiertem Streptavidin markierten sekundären Antikörpern oder alternativ unter Verwendung von mit Peroxidase oder direkt mit Farbstoffen konjugierten Dextranpolymeren quantifiziert wird.

5. Prüfung gemäß Anspruch 4, wobei die Expression auf einem oder mehreren Schnitten der Probe mit Nachweistechniken unter Verwendung von sekundären Antikörpern, die mit einem unterschiedlichen Fluorochrom für jeden sekundären Antikörper markiert sind, quantifiziert wird.

6. Prüfung gemäß einem der Ansprüche 1 oder 2, wobei die Expression durch Nachweisen von mRNA für jeden der Marker quantifiziert wird.

7. Prüfung gemäß einem der Ansprüche 1 bis 6, wobei die Biopsieprobe eine in Paraffin eingeschlossene Probe ist.

8. Verfahren zum Vorhersagen der Ansprechempfindlichkeit oder Nicht-Ansprechempfindlichkeit von Patienten mit massiven Tumoren auf eine Behandlung durch Immuntherapie, optional in Kombination mit Strahlentherapie oder Chemotherapie oder Target-Therapie, das das Bestimmen der Tumormikroumgebung mit der Prüfung, wie in einem der Ansprüche 1 bis 7 beschrieben, beinhaltet, wobei Patienten, die eine immunogene Tumormikroumgebung aufweisen, auf eine Immuntherapiebehandlung ansprechen werden, während Patienten, die eine immunsuppressive Tumormikroumgebung aufweisen, nicht auf eine Behandlung durch Immuntherapie ansprechen werden.

9. Verfahren gemäß Anspruch 8, wobei die Immuntherapie mit einem oder mehreren immunologischen Checkpoint-Inhibitor-Medikamenten durchgeführt wird.

10. Kit zum Beurteilen der Tumormikroumgebung, umfassend:
- Reagenzien zum Quantifizieren der Expression des hMENA11a-Markers in einer Biopsieprobe;
- Reagenzien zum Quantifizieren der Expression des FN1-Markers in einer Biopsieprobe;
- Reagenzien zum Quantifizieren der Expression der CD3-, CD8- und CD20-Marker in einer Biopsieprobe; und
- Reagenzien zum Quantifizieren der Expression des POL1-Markers in einer Biopsieprobe.

11. Kit gemäß Anspruch 10, ferner umfassend Reagenzien zum Quantifizieren der Expression des hMena-ΔV6-Markers und/oder Reagenzien zum Quantifizieren der Expression des gesamten hMena-Markers, das heißt aller hMena-Proteinisoformen in einer Biopsieprobe.

12. Kit gemäß einem der Ansprüche 10 oder 11, wobei die Reagenzien spezifische Antikörper umfassen, die selektiv die 11a-lsoform von hMENA des hMena-Proteins binden, spezifische Antikörper, die selektiv CD3 binden, spezifische Antikörper, die selektiv CD8 binden, spezifische Antikörper, die selektiv CD20 binden, und spezifische Antikörper, die selektiv FN1 binden, und optional mit spezifischen Antikörpern, die selektiv die hMena-ΔV6-Isoform des hMena-Proteins binden, und/oder Anti-Gesamt-hMena-spezifischen Antikörpern, das heißt Antikörpern, die alle Isoformen des hMena-Proteins binden.

13. Kit gemäß Anspruch 12 ferner umfassend sekundäre Antikörper, die mit Peroxidase-konjugiertem Streptavidin markiert sind oder alternativ Dextranpolymere umfassen, die mit Peroxidase oder direkt mit Farbstoffen konjugiert sind.

14. Kit gemäß Anspruch 12 ferner umfassend sekundäre Antikörper, die mit einem unterschiedlichen Fluorochrom für jeden sekundären Antikörper markiert sind.

15. Kit gemäß einem der Ansprüche 10 oder 11, wobei die Reagenzien Oligonukleotide sind, die spezifisch für den Nachweis der mRNA jedes der Marker sind.

## Revendications

1. Dosage pour déterminer le microenvironnement, immunogénique ou immunodépresseur, d'une tumeur, comprenant la quantification, dans un échantillon biopsique de ladite tumeur, de :
- l'expression du marqueur hMENA11a dans des cellules tumorales et dans les cellules stromales dudit échantillon ;
- l'expression du marqueur FN1 dans le stroma dudit échantillon ;
- l'expression des marqueurs CD3, CD8 et CD20 dans les cellules dudit échantillon ; et
- l'expression du marqueur PDL1 dans les cellules tumorales dudit échantillon,
dans lequel
l'expression de hMENA11a supérieure à une valeur seuil de 50, identifiée par analyse ROC, dans les cellules tumorales dudit échantillon ; l'absence d'expression de hMENA11a dans les cellules stromales dudit échantillon ; l'expression du marqueur FN1 inférieure à une valeur d'intensité, appelée score, égale à 2 dans une échelle d'intensité de coloration, de 0 à 3 dans le stroma dudit échantillon et l'expression des marqueurs CD3, CD8 et CD20 dans au moins 5 cellules par mm² dans la zone tumorale ou dans la marge d'invasion dudit échantillon ; la présence de structure lymphoïdes tertiaires (TLS) consistant en un agrégat de lymphocytes B à l'intérieur de la structure et de cellules T à l'extérieur de celle-ci, déterminés respectivement par l'expression de marqueurs CD20, CD8 et CD3, sont représentatives d'un microenvironnement tumoral immunogénique, tandis que
l'expression de hMENAU1a inférieure à une valeur seuil de 50 dans les cellules tumorales dudit échantillon ; l'expression du marqueur FN1 égale ou supérieure à une valeur seuil de score de 2 dans le stroma dudit échantillon et l'absence d'expression des marqueurs CD3, CD8 et CD20 dans des cellules à l'intérieur de la zone tumorale ou dans la marge d'invasion dudit échantillon, sont représentatives d'un microenvironnement tumoral immunodépresseur ;
dans lequel :
hMENA11a est le variant d'ENAH 1 ID : NM_001008493 qui est un variant d'épissage contenant un exon additionnel (11a) ;
hMENADv6 est un variant d'épissage de hMENA dépourvu de l'exon 6, ID : EU255274.1 ;
FN1 est le gène de fibronectine 1, ID : 2335 ;
PD-L1 est le gène du ligand 1 de mort cellulaire programmée, alias CD274, ID : 29126.

2. Dosage selon la revendication 1, dans lequel l'expression du marqueur hMena □V6 et/ou du marqueur hMena totale, dans lequel ledit marqueur hMena totale comprend toutes les isoformes de la protéine hMena, est en outre quantifiée dans les cellules tumorales et stromales dudit échantillon, dans lequel :
l'absence d'expression de hMena □V6 dans des cellules tumorales dudit échantillon et/ou l'expression de hMena totale inférieure à une valeur seuil de 80 dans les cellules tumorales dudit échantillon ; et l'absence de n'importe lesquels desdits marqueurs dans le stroma dudit échantillon, sont représentatives d'un microenvironnement tumoral immunogénique ; tandis que
la présence de l'expression de hMena □V6 dans des cellules tumorales dudit échantillon et une expression de celui-ci dans les cellules stromales dudit échantillon, et/ou
la présence de l'expression de hMena totale supérieure à une valeur seuil de 80 dans les cellules tumorales et l'expression de celui-ci dans les cellules stromales dudit échantillon, sont représentatives d'un microenvironnement tumoral immunodépresseur.

3. Dosage selon l'une quelconque des revendications 1 et 2, dans lequel ladite expression est quantifiée par des techniques immunohistochimiques avec des anticorps spécifiques qui se lient sélectivement à l'isoforme 11a de hMENA de la protéine hMena, des anticorps spécifiques qui se lient sélectivement à CD3, des anticorps spécifiques qui se lient sélectivement à CD8, des anticorps spécifiques qui se lient sélectivement à CD20 et des anticorps spécifiques qui se lient sélectivement à FN1 et, éventuellement, des anticorps spécifiques qui se lient sélectivement à l'isoforme hMena □V6 de la protéine hMena et/ou des anticorps spécifiques anti-hMena totale, c'est-à-dire des anticorps qui se lient à toutes les isoformes de la protéine hMena.

4. Dosage selon la revendication 3, dans lequel ladite expression est quantifiée sur des sections consécutives dudit échantillon, chacune pour chaque marqueur, avec des techniques de détection utilisant des anticorps secondaires marqués avec de la streptavidine conjuguée à de la peroxydase, ou en variante utilisant des polymères de dextrane conjugués à de la peroxydase ou directement à des colorants.

5. Dosage selon la revendication 4, dans lequel ladite expression est quantifiée sur une ou plusieurs sections dudit échantillon, avec des techniques de détection utilisant des anticorps secondaires marqués avec un fluorochrome différent pour chaque anticorps secondaire.

6. Dosage selon l'une quelconque des revendications 1 et 2, dans lequel ladite expression est quantifiée par détection d'ARNm de chacun desdits marqueurs.

7. Dosage selon l'une quelconque des revendications 1 à 6, dans lequel ledit échantillon de biopsie est un échantillon inclus dans de la paraffine.

8. Procédé pour prédire la sensibilité ou l'insensibilité, de patients ayant des tumeurs solides, à un traitement par immunothérapie éventuellement en combinaison avec une radiothérapie ou une chimiothérapie ou une thérapie ciblée, qui comprend la détermination du microenvironnement tumoral avec le dosage tel que décrit dans l'une quelconque des revendications 1 à 7, dans lequel des patients présentant un microenvironnement tumoral immunogénique seront sensibles à un traitement par immunothérapie tandis que les patients présentant un microenvironnement tumoral immunodépresseur seront insensibles à un traitement par immunothérapie.

9. Procédé selon la revendication 8, dans lequel ladite immunothérapie est effectuée avec un ou plusieurs médicaments inhibiteurs de point de contrôle immunologique.

10. Trousse pour la détermination du microenvironnement d'une tumeur, comprenant :
- des réactifs pour quantifier l'expression du marqueur hMENA11a dans un échantillon de biopsie ;
- des réactifs pour quantifier l'expression du marqueur FN1 dans un échantillon de biopsie ;
- des réactifs pour quantifier l'expression des marqueurs CD3, CD8 et CD20 dans un échantillon de biopsie ; et
- des réactifs pour quantifier l'expression du marqueur PDL1 dans un échantillon de biopsie.

11. Trousse selon la revendication 10, comprenant en outre des réactifs pour quantifier l'expression du marqueur hMena □V6 et/ou des réactifs pour quantifier l'expression du marqueur hMena totale, c'est-à-dire toutes les isoformes de la protéine hMena, dans un échantillon de biopsie.

12. Trousse selon l'une quelconque des revendications 10 et 11, dans laquelle lesdits réactifs comprennent des anticorps spécifiques qui se lient sélectivement à l'isoforme 11a de hMENA de la protéine hMena, des anticorps spécifiques qui se lient sélectivement à CD3, des anticorps spécifiques qui se lient sélectivement à CD8, des anticorps spécifiques qui se lient sélectivement à CD20 et des anticorps spécifiques qui se lient sélectivement à FN1 et, éventuellement, des anticorps spécifiques qui se lient sélectivement à l'isoforme hMena □V6 de la protéine hMena et/ou des anticorps spécifiques anti-hMena totale, c'est-à-dire des anticorps qui se lient à toutes les isoformes de la protéine hMena.

13. Trousse selon la revendication 12, comprenant en outre des anticorps secondaires marqués avec de la streptavidine conjuguée à de la peroxydase, ou en variante comprenant des polymères de dextrane conjugués à de la peroxydase ou directement à des colorants.

14. Trousse selon la revendication 12, comprenant en outre des anticorps secondaires marqués avec un fluorochrome différent pour chaque anticorps secondaire.

15. Trousse selon l'une quelconque des revendications 10 et 11, dans laquelle lesdits réactifs sont des oligonucléotides spécifiques de la détection de l'ARNm de chacun desdits marqueurs.
